(19) **Europäisches Patentamt / European Patent Office / Office européen des brevets**

(11) **EP 3 833 265 B1**

(12) **EUROPEAN PATENT SPECIFICATION**

(45) Date of publication and mention of the grant of the patent:
**09.03.2022 Bulletin 2022/10**

(21) Application number: **19752676.7**

(22) Date of filing: **07.08.2019**

(51) International Patent Classification (IPC):
**A61B 8/08** (2006.01)

(52) Cooperative Patent Classification (CPC):
**A61B 8/0841; A61B 8/5207; A61B 90/37; G01S 3/803; G01S 5/28;** A61B 8/12; A61B 8/461; A61B 8/5246; A61B 8/5269; A61B 8/58; A61B 2017/3413; A61B 2034/2063; A61B 2090/378; A61B 2090/3782; A61B 2090/3784; (Cont.)

(86) International application number:
**PCT/EP2019/071163**

(87) International publication number:
**WO 2020/030665 (13.02.2020 Gazette 2020/07)**

(54) **INTERVENTIONAL DEVICE POSITIONING RESPECTIVE AN ULTRASOUND IMAGE PLANE**

POSITIONIERUNG EINER INTERVENTIONELLEN VORRICHTUNG IN BEZUG AUF EINE ULTRASCHALLBILDEBENE

POSITIONNEMENT D'UN DISPOSITIF D'INTERVENTION PAR RAPPORT À UN PLAN D'IMAGE ULTRASONORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2018 US 201862716115 P**
**05.10.2018 EP 18198814**

(43) Date of publication of application:
**16.06.2021 Bulletin 2021/24**

(73) Proprietor: **Koninklijke Philips N.V.**
**5656 AG Eindhoven (NL)**

(72) Inventors:
- **MEGENS, Mischa**
  **5656 AE Eindhoven (NL)**
- **STAPERT, Hendrik, Roelof**
  **5656 AE Eindhoven (NL)**
- **GOKGURLER, Mustafa, Hakan**
  **5656 AE Eindhoven (NL)**
- **VAN DE PAS, Stefan**
  **5656 AE Eindhoven (NL)**
- **KORTSMIT, Jeroen**
  **5656 AE Eindhoven (NL)**
- **VAN HEESCH, Franciscus, Hendrikus**
  **5656 AE Eindhoven (NL)**
- **BELT, Harm, Jan, Willem**
  **5656 AE Eindhoven (NL)**
- **JAIN, Ameet, Kumar**
  **5656 AE Eindhoven (NL)**
- **VAIDYA, Kunal**
  **5656 AE Eindhoven (NL)**
- **VIGNON, Francois, Guy, Gerard, Marie**
  **5656 AE Eindhoven (NL)**

(74) Representative: **Philips Intellectual Property & Standards**
**High Tech Campus 52**
**5656 AG Eindhoven (NL)**

(56) References cited:
WO-A1-2018/087111 WO-A1-2018/108717
US-A- 4 249 539 US-A1- 2005 038 341
US-A1- 2010 298 704 US-A1- 2016 324 501
US-A1- 2017 027 605

(52) Cooperative Patent Classification (CPC): (Cont.)
A61B 2090/3786; A61B 2090/3788

**Description**

FIELD OF THE INVENTION

The invention relates to determining a position of an interventional device respective an image plane of a beamforming ultrasound imaging probe.

BACKGROUND OF THE INVENTION

Interventional devices such as medical needles, catheters and surgical tools are often difficult to visualize in an ultrasound image due to the specular nature of their reflectivity, particularly at unfavorable incidence angles.

In this respect, document WO2018060499A1 describes a system for indicating a position of an interventional device feature of an interventional device respective an image plane defined by an ultrasound imaging probe of a beamforming ultrasound imaging system in which the position of the interventional device feature is determined based on ultrasound signals transmitted between the ultrasound imaging probe and an ultrasound transducer attached to the interventional device at a predetermined distance from the interventional device feature. An icon providing unit provides a first icon indicative of a circular zone with a radius corresponding to the predetermined distance. The first icon is displayed in a fused image that includes a reconstructed ultrasound image from the beamforming ultrasound imaging system.

Documents US 2016/0324501 A1, WO2011138698A1, WO2015101949A1 and WO2016009350A1 also describe systems for tracking an instrument in an ultrasound field with an ultrasound receiver that is mounted to the instrument. The position of the ultrasound receiver is subsequently displayed in an ultrasound image corresponding to the ultrasound field.

Despite these solutions there remains room for improved techniques for determining a position of an interventional device respective an ultrasound imaging plane.

SUMMARY OF THE INVENTION

The invention is defined in the appended independent claims. Preferred embodiments are defined in the dependent claims.

In seeking to improve the positioning of an interventional device respective an image plane of a beamforming ultrasound imaging probe, a system is provided for determining a position of an interventional device respective an image plane defined by an ultrasound imaging probe of a beamforming ultrasound imaging system in which the position of the interventional device is determined based on ultrasound signals transmitted between the ultrasound imaging probe and an ultrasound transducer attached to the interventional device. The system includes an image reconstruction unit and a position determination unit. The image reconstruction unit provides a reconstructed ultrasound image corresponding to an image plane defined by the ultrasound imaging probe. The position determination unit computes a position of the ultrasound transducer respective the image plane based on a time of flight of a maximum detected intensity ultrasound signal transmitted between the ultrasound imaging probe and the ultrasound transducer; and indicates the computed position in the reconstructed ultrasound image. Moreover, the position determination unit suppresses the indication of the computed position if at least one of the following conditions are met:

>   a rate of change of the computed position exceeds a first predetermined rate;
>   an interference signal in the ultrasound signals exceeds a first predetermined value.

An issue that has been discovered by the inventors of the present invention is that the position determined by the position determination unit may be susceptible to errors. The likelihood of a potentially inaccurate position can be reliably determined by monitoring the aforementioned parameters. By suppressing the indication of the computed position under the aforementioned conditions, it is prevented that a potentially inaccurate position is indicated.

In accordance with one aspect the position determination unit continues suppressing the indication of the computed position until at least one of the following corresponding conditions have been satisfied for predetermined period:

>   a rate of change of the computed position is less than a second predetermined rate;
>   an interference signal in the ultrasound signals is less than a second predetermined value.

The second predetermined rate, value, threshold and level may be equal to or different from the corresponding first predetermined parameters. By continuing to suppress the indication of the computed position until the corresponding condition is met for a predetermined period, the reliability of the system is further improved because it is ensured that the computed position is not indicated again until the position has stabilized over time. The optional use of a different threshold adds hysteresis into the decision making. In so doing more reliable system is provided.

In accordance with one aspect the condition for suppressing indication of the computed position is based on an interference signal in the ultrasound signals exceeding a first predetermined value. The position determination unit measures the interference and/ or noise signal in the ultrasound signals between consecutive imaging frame periods. Between consecutive frame periods there is a "quiet" period during which negligible ultrasound signals are expected to be transmitted by the ultrasound imaging probe and no reflected ultrasound signals are expected to be detected. Consequently this quiet period represents a time when only the interference and/ or noise detected by the system can be reliably measured.

In accordance with one aspect the condition for suppressing indication of the computed position is based on an interference signal in the ultrasound signals exceeding a first predetermined value. The position determination unit measures the interference and/ or noise signal in the ultrasound signals between consecutive image line periods. Image lines are typically transmitted consecutively, and include a transmit phase and a receive phase during which the ultrasound imaging probe transmits ultrasound signals and subsequently receives reflected ultrasound signals. Between the end of the receive phase of one imaging line and the transmit phase of the subsequent image line, there is a "quiet" period in which no reflections are expected. Consequently this quiet period represents a time when only the interference and/ or noise detected by the system can be reliably measured.

In accordance with other aspects a method and corresponding computer program product that may be used in conjunction with the system are provided.

It is to be noted that the various aspects described in relation to the system may be combined to provide further advantageous effects. Moreover, aspects of the system may be used interchangeably with the method, and vice versa.

BRIEF DESCRIPTION OF THE FIGURES

Fig. 1 illustrates a beamforming ultrasound imaging system 14 in combination with an in-plane interventional device 11 and an embodiment of the invention in the form of system 10.

Fig. 2 illustrates a beamforming ultrasound imaging system 14 in combination with an interventional device 11 disposed at an out-of-plane distance $D_{op}$ and an embodiment of the invention in the form of system 10.

Fig. 3 illustrates a reconstructed ultrasound image RUI in which a computed position $LAP_{TOFSmax, \theta IPA}$ of the interventional device is indicated.

Fig. 4 illustrates a succession of periodic updates, RUI', RUI" to reconstructed ultrasound image RUI.

Fig. 5 illustrates a reconstructed ultrasound image RUI that includes multiple image lines $L_{1..n}$, each line corresponding to a time of flight or depth dimension in the ultrasound image.

Fig. 6 illustrates a model MO describing an expected variation of in-plane maximum detected intensity, $I_{SmaxInplane}$ (dB) with time of flight, TOF.

Fig. 7 illustrates a succession of reconstructed ultrasound images RUI in which an out-of-plane distance $D_{op}$ is indicated by means of a first icon $C_{op}$ at the computed lateral position $LAP_{TOFSmax, \theta IPA}$.

Fig. 8 illustrates an interventional device 11 that is suitable for use with system 10.

Fig. 9 illustrates various method steps of a method that may be used with system 10.

DETAILED DESCRIPTION OF THE INVENTION

In order to illustrate the principles of the present invention, various systems are described in which the position of an interventional device, exemplified by a medical needle, is indicated respective an image plane defined by a linear array of a 2D ultrasound imaging probe. Moreover, in some examples the position of a feature, such as the distal end, of the medical device is also tracked.

It is however to be appreciated that the invention also finds application with other interventional devices such as, and without limitation, a catheter, a guidewire, a probe, an endoscope, an electrode, a robot, a filter device, a balloon device, a stent, a mitral clip, a left atrial appendage closure device, an aortic valve, a pacemaker, an intravenous line, a drainage line, a surgical tool, a tissue sealing device, a tissue cutting device or an implantable device. The tracked feature of such interventional devices may exemplarily include a distal end of the interventional device, a biopsy sampling point of the interventional device, a cutting edge of the interventional device, an opening of a channel in the interventional device, a sensor (e.g. for sensing flow, pressure, temperature etc.) of the interventional device, a surgical tool (e.g. a scraper) integrated in the interventional device, a drug delivery point of the interventional device, or an energy delivery point of the interventional device.

Furthermore it is to be appreciated that the exemplified linear array of a 2D ultrasound imaging probe is only one example of an ultrasound transceiver array of a beamforming ultrasound imaging system in which the invention may be used. The invention also finds application in other types of beamforming ultrasound imaging systems whose associated ultrasound transceiver arrays exemplarily include a 2D array of a 3D imaging probe (or in bi-plane view), a "TRUS" transrectal ultrasonography probe, an "IVUS" intravascular ultrasound probe, a "TEE" transesophageal probe, a "TTE" transthoracic probe, a "TNE" transnasal probe, an "ICE" intracardiac probe.

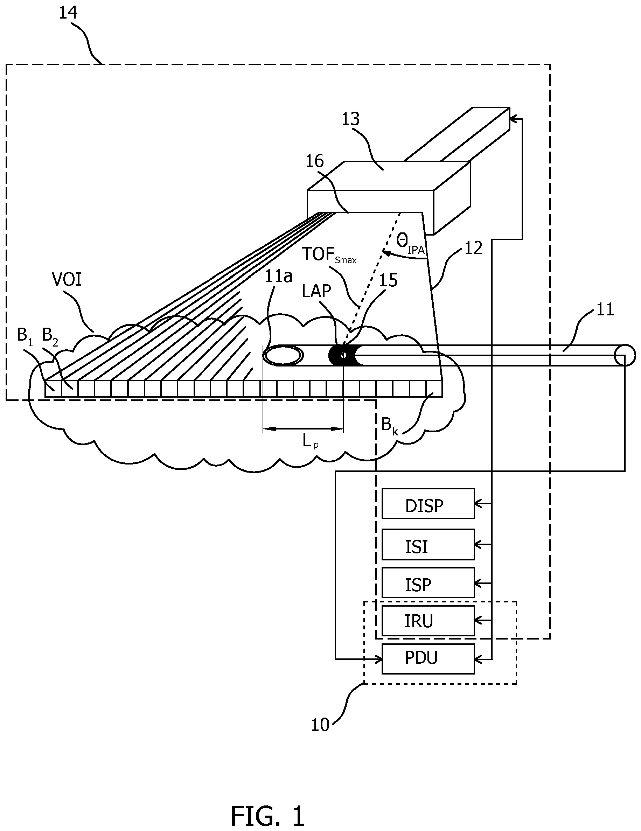

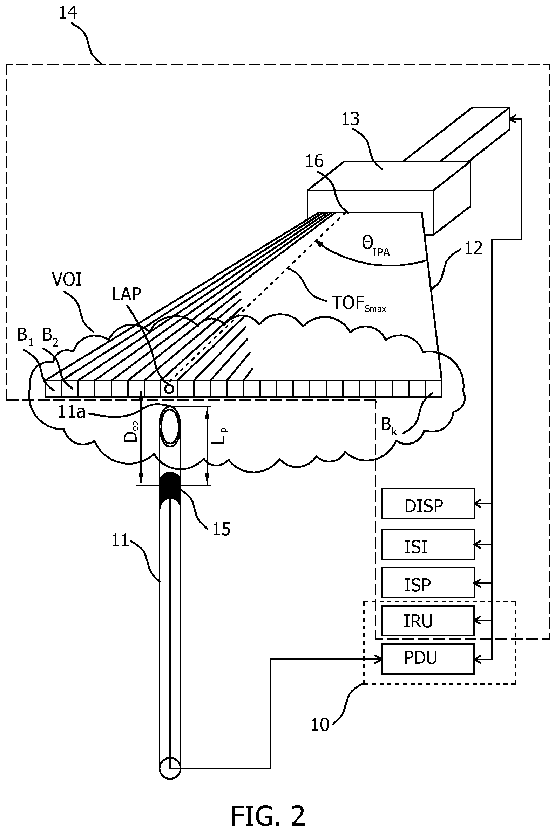

Fig. 1 illustrates a beamforming ultrasound imaging system 14 in combination with an in-plane interventional device 11 and an embodiment of the invention in the form of system 10. In Fig. 1, beamforming ultrasound imaging system 14 includes a 2D ultrasound imaging probe 13 which is in communication with image reconstruction unit IRU, imaging system processor ISP, imaging system interface ISI and display DISP. The units IRU, ISP, ISI and DISP are conventionally located in a console that is in wired communication with 2D ultrasound imaging probe 13. It is also contemplated that wireless communication, for example using an optical, infrared, or an RF communication link, may replace the wired link. It is also contemplated that some of units IRU, ISP, ISI and DISP may instead be incorporated within 2D ultrasound imaging probe 13, as in for example the Philips Lumify ultrasound imaging system. In Fig. 1, 2D ultrasound imaging probe 13 includes linear ultrasound transceiver array 16 that transmits and receives ultrasound energy within an ultrasound field that intercepts volume of interest VOI. The ultrasound field is fan-shaped in Fig. 1 and includes multiple ultrasound beams $B_{1..k}$ that define image plane 12. Note that a fan-shaped beam is illustrated in Fig. 1 for the purposes of illustration only and that the invention is not limited to a particular shape of ultrasound field. Beamforming ultrasound imaging system 14 may also include electronic driver and receiver circuitry, not shown, that is configured to amplify and/ or to adjust the phase of signals transmitted by or received by 2D ultrasound imaging probe 13 in order to generate and detect ultrasound signals in beams $B_{1..k}$. The electronic driver and receiver circuitry may thus be used to steer the emitted and/ or received ultrasound beam direction.

In-use, beamforming ultrasound imaging system 14 is operated in the following way. An operator may plan an ultrasound procedure via imaging system interface ISI. Once an operating procedure is selected, imaging system interface ISI triggers imaging system processor ISP to execute application-specific programs that generate and interpret the signals transmitted by and detected by 2D ultrasound imaging probe 13. Beamforming ultrasound imaging system 14 may also include a memory, not shown, for storing such programs. The memory may for example store ultrasound beam control software that is configured to control the sequence of ultrasound signals transmitted by and/ or received by ultrasound imaging probe 13. Image reconstruction unit IRU, which may alternatively form part of imaging system processor ISP, reconstructs data received from the ultrasound imaging probe 13 into an image corresponding to image plane 12 and which thus intercepts volume of interest VOI, and subsequently displays this image on display DISP. A planar section through volume of interest VOI is termed region of interest ROI herein. Reconstructed ultrasound image RUI may thus include region of interest ROI. The reconstructed image may for example be an ultrasound Brightness-mode "B-mode" image, otherwise known as a "2D mode" image, a "C-mode" image or a Doppler mode image, or indeed any ultrasound planar image.

Also shown in Fig. 1 is a medical needle 11 as an example of an interventional device, and an embodiment of the invention, system 10, that may be used to indicate a position of interventional device 11, i.e. the medical needle, respective image plane 12 of ultrasound imaging probe 13. This embodiment, system 10, includes image reconstruction unit IRU and position determination unit PDU. These units are in communication with one another as illustrated by the interconnecting arrows. It is also contemplated that one or more of units PDU, IRU may be incorporated within a memory or a processor of beamforming ultrasound imaging system 14, for example within a memory or a processor that also provides the functionality of unit ISP. Medical needle 11 that is tracked, includes ultrasound transducer 15 that may be positioned at predetermined distance $L_p$ from distal end 11a of interventional device 11.

In-use, a position of interventional device 11, or more specifically that of ultrasound transducer 15 attached thereto, is computed respective image plane 12 by position determination unit PDU based on ultrasound signals transmitted between ultrasound transceiver array 16 and ultrasound transducer 15.

In one configuration ultrasound transducer 15 is a detector that receives ultrasound signals corresponding to beams $B_{1..k}$. Position determination unit PDU identifies the lateral position LAP of ultrasound transducer 15 respective image plane 12 by correlating; i.e. comparing, the ultrasound signals emitted by ultrasound transceiver array 16 with the ultrasound signals detected by ultrasound transducer 15. More specifically this correlation determines the best fit position of ultrasound transducer 15 respective image plane 12 based on i) the intensities of the ultrasound signals corresponding to each beam $B_{1..k}$ that are detected by ultrasound transducer 15 and ii) based on the time delay, i.e. time of flight, between emission of each beam $B_{1..k}$ and its detection by ultrasound transducer 15. This may be illustrated as follows. When ultrasound transducer 15 is in the vicinity of image plane 12, ultrasound signals from the nearest of beams $B_{1..k}$ to the transducer will be detected with a relatively larger intensity whereas more distant beams will be detected with relatively smaller intensities. Typically the beam that is detected with the maximum detected intensity is identified as the one that is closest to ultrasound detector 15. In other words, the maximum detected intensity $I_{Smax}$ ultrasound signal identifies the in-plane angle $\Theta_{IPA}$ between ultrasound transceiver array 16 and ultrasound transducer 15. The time of flight, between the emission of this beam (from beams $B_{1..k}$) and its subsequent detection is indicative of the range between ultrasound transceiver array 16 and ultrasound transducer 15. Thus the time delay of the ultrasound signal in the beam that was detected with maximum detected intensity, $I_{Smax}$, i.e. TOFsmax, is the ultrasound signal that is selected from the ultrasound signals of all beams. Since the time of flight is indicative of the range, in polar coordinates the lateral position of ultrasound transducer 15 respective image plane 12 may be represented by $LAP_{TOFSmax,\ \theta IPA}$. If desired, the range may be determined by multiplying the time delay by the speed of ultrasound propagation.

In another configuration ultrasound transducer 15 is an emitter that emits one or more ultrasound pulses. Such pulses may for example be emitted during tracking frames that are interleaved between the usual imaging frames of ultrasound imaging system 14. In such a tracking frame the ultrasound transceiver array 16 may be operated in a receive-only mode in which it listens for ultrasound signals originating from the vicinity of image plane 12. Ultrasound transceiver array 16 is thus configured as a one-way receive-only beamformer. Position determination unit PDU identifies from which beam of beams $B_{1..k}$ the pulse(s) originated based on the ultrasound signals emitted by ultrasound transducer 15 and those detected by ultrasound transceiver array 16. As in the configuration above, position determination unit PDU may use a correlation procedure that, based on the ultrasound signal detected with maximum intensity and its time of flight, identifies the closest beam and thus the point at which the ultrasound signal was emitted, i.e. its lateral position $LAP_{TOFSmax,\ \theta IPA}$ in the same manner. Thus, when ultrasound transducer 15 is an emitter, a correlation, i.e. comparison, procedure may again be used to determine its best-fit position respective image plane 12 for each tracking frame.

In another configuration ultrasound transducer 15 may be configured to act as both a receiver and an emitter, or include both a receiver and an emitter. In this configuration ultrasound transducer 15 may be triggered to emit one or more ultrasound pulses upon receipt of an ultrasound signal from ultrasound transceiver array 16; optionally following a delay that is equal to one or more frame periods of ultrasound imaging system 14. In this way the pulse(s) emitted by ultrasound transducer 15 during an imaging mode are received by ultrasound transceiver array 16 in the form of an echo in the reconstructed ultrasound at an in-plane angular position, i.e. in an image line, that corresponds to the triggering beam $B_{1..k}$. Ultrasound transducer 15 thus appears as a bright spot in the reconstructed image. Position determination unit PDU may subsequently identify this bright spot in the reconstructed image and thus again compute a lateral position $LAP_{TOFSmax,\ \theta IPA}$ of ultrasound transducer 15 respective image plane 12.

In yet another configuration, not illustrated, ultrasound imaging probe 13 may further include at least three ultrasound emitters that are attached to the ultrasound imaging probe 13. The at least three ultrasound emitters are in communication with position determination unit PDU. Moreover the position determination unit PDU is configured to compute a position of the ultrasound transducer 15 respective the image plane 12 based on ultrasound signals transmitted between the at least three ultrasound emitters attached to the ultrasound imaging probe 13, and the ultrasound transducer 15. In this configuration position determination unit PDU determines a range between each emitter and ultrasound transducer 15 based on the time of flight of ultrasound signals emitted by each emitter. The three dimensional position of ultrasound transducer 15 is subsequently determined using triangulation. This provides the position of ultrasound transducer 15 in three dimensions respective ultrasound imaging probe 13, or more specifically respective image plane 12 since the at least three emitters are attached to the ultrasound imaging probe 13. The three-dimensional position may subsequently be mapped to image plane 12 and thus again represented by $LAP_{TOFSmax,\ \theta IPA}$. Ultrasound emitters are preferred in this configuration because the supply of high power ultrasound signals to the emitters, necessary for accurate positioning over a large range, is simpler when the emitters are proximate ultrasound imaging probe 13 where a power source is readily available. This arrangement is thus preferred in contrast to locating a high power emitter on interventional device 11. In-use, the lateral position of interventional device 11, or more specifically that of ultrasound transducer 15 attached thereto, is thus again computed respective image plane 12 by position determination unit PDU based on ultrasound signals transmitted between the at least three emitters and ultrasound transducer 15.

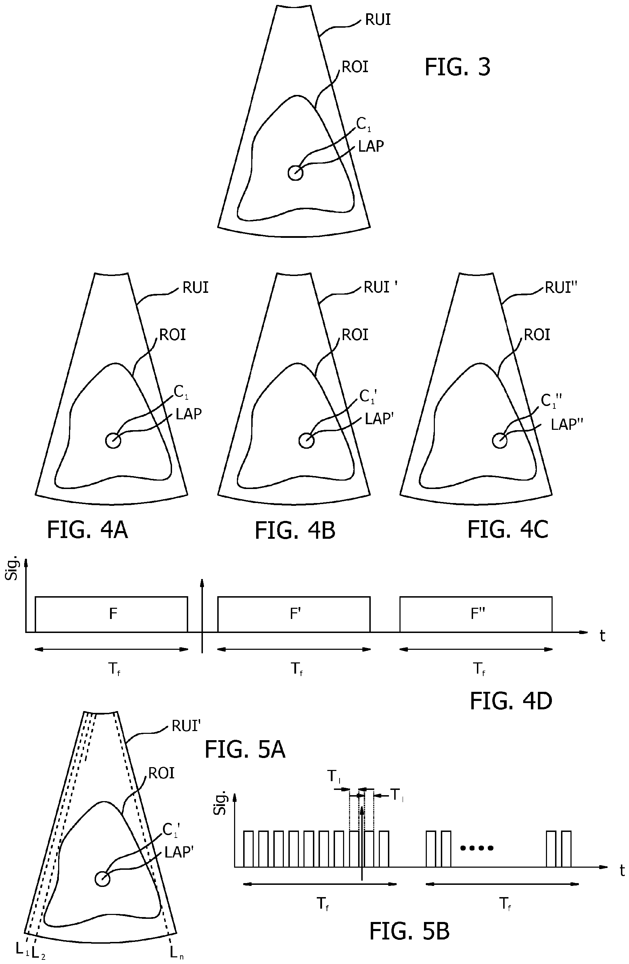

In summary, in this in-plane arrangement in which ultrasound transducer 15 is in the image plane, position determination unit PDU illustrated in Fig. 1 may be used in any of the above configurations to compute a lateral position of ultrasound transducer 15 respective image plane 12 based on ultrasound signals transmitted between ultrasound imaging probe 13 and ultrasound transducer 15. With reference to Fig. 3, which illustrates a reconstructed ultrasound image RUI in which a computed position $LAP_{TOFSmax,\ \theta IPA}$ of the interventional device is indicated, after computation of the position, computed position $LAP_{TOFSmax,\ \theta IPA}$ may be indicated in reconstructed ultrasound image RUI. Position LAP may for example be indicated as shown by the exemplary circle $C_1$, the center of which corresponds to computed position $LAP_{TOFSmax,\ \theta IPA}$. Alternative icons, shapes and indications may likewise be used. Whilst a circle is indicated in Fig. 3, other icons than a complete circle and which are likewise indicative of a circular zone may be used in the same manner, including e.g. a circular arrangement of dots or dashes, a circular arrangement of radiallydirected lines or arrows, the tips of which indicate a circular zone, and so forth. In the exemplified circle in Fig. 3, the perimeter of the circle may indicate the limit of the uncertainty of position LAP, or a range of possible positions of e.g. a feature that is disposed on interventional device 11 at a predetermined distance from ultrasound transducer 15.

When ultrasound transducer 15 is disposed away from the image plane, i.e. out-of-plane, the same procedure may be used to determine a lateral position of ultrasound transducer 15, i.e. a position projected onto image plane 12. An additional procedure that uses the intensity, Ismax, and the time of flight, TOFsmax, of the ultrasound signal having the maximum detected intensity, may optionally be used to estimate a distance of ultrasound transducer 15 from image plane 12. In this respect, Fig. 2 illustrates a beamforming ultrasound imaging system 14 in combination with an interventional device 11 disposed at an out-of-plane distance $D_{op}$ and an embodiment of the invention in the form of system 10. Although beams $B_{1.k}$ of ultrasound imaging probe 13 are illustrated as being in plane 12, this plane has a finite thickness and a reduced ultrasound signal is typically detectable for small out-of-plane displacements. These signals are used in the present invention to estimate the out-of-plane distance $D_{op}$ of ultrasound transducer 15.

Various techniques may be used to determine the out-of-plane distance $D_{op}$. One technique involves using a transducer-specific three-dimensional map that associates a three dimensional position respective image plane 12 to an expected signal intensity. Having determined the lateral position $LAP_{TOFSmax, \theta IPA}$ of ultrasound transducer 15 respective image plane 12 as described above, the out of plane distance is determined by looking-up in the model the out of plane distance corresponding to the detected intensity, Ismax, at that lateral position.

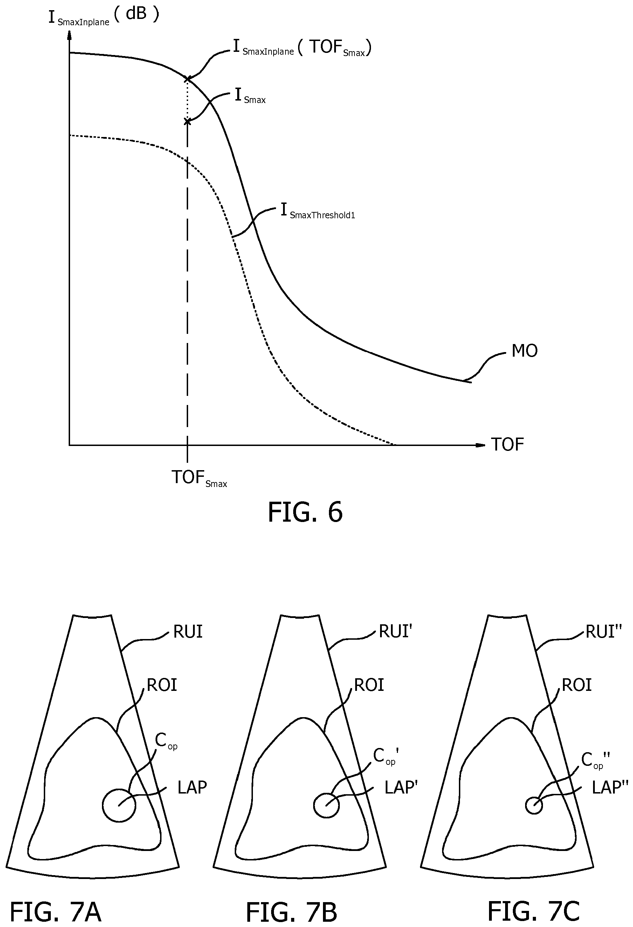

Another technique is now described with reference to Fig. 6, which illustrates a model MO describing an expected variation of in-plane maximum detected intensity, $I_{SmaxInplane}$ (dB) with time of flight, TOF. With reference to Fig. 6, Model MO, indicated by the solid curve, illustrates that as the time of flight TOF, i.e. the depth into tissue increases, the in-plane maximum detected intensity, $I_{SmaxInplane}$, of detected ultrasound signals initially decreases slowly, then more rapidly, and then more slowly again. The shape of the model is affected by attenuation of ultrasound signals and may be determined from theoretical calculations or empirical measurements of the in-plane maximum intensity obtained in tissue or corresponding matter. Model MO depends only on time of flight and is invariant with in-plane angle $\theta_{IPA}$. It is noted that model MO does not model the maximum detected intensity, $I_{SmaxInplane}$ as a function of out-of-plane distance. Consequently model MO requires only a limited amount of, i.e. one-dimensional, calibration data. In contrast to the aforementioned three-dimensional model, in-use the out-of-plane distance may be determined with model MO with low latency due to the need to search in only one, i.e. time of flight, dimension. The modeled in-plane maximum detected intensity, $I_{SmaxInplane}$ has been found to reliably represent different beamforming ultrasound imaging probes of the same type, which means that the same model may be used for beamforming ultrasound imaging probes of the same type.

With reference to Fig. 2 and Fig. 3, in-use, computing out-of-plane distance $D_{op}$ comprises comparing the maximum detected intensity Ismax with model MO. The out-of-plane distance $D_{op}$ may subsequently be indicated in reconstructed ultrasound image RUI. The out-of-plane distance may be indicated numerically for example, or as a size, or a color of an icon that varies accordance with $D_{op}$. With reference to Fig. 3, in one implementation the out-of-plane distance $D_{op}$ may be indicated by means of varying the radius of circle $C_1$ in Fig. 3 in accordance with out-of-plane distance $D_{op}$. Comparing the maximum detected intensity Ismax with model MO may for instance involve determining a difference or ratio between detected intensity $I_{Smax}$ and the in-plane maximum detected intensity, $I_{SmaxInplane}$ at the time of flight TOFsmax corresponding to the computed lateral position $LAP_{TOFSmax}$. In one exemplary implementation the maximum detected intensity $I_{Smax}$ at the computed lateral position $LAP_{TOFSmax, \theta IPA}$ of the ultrasound transducer may thus be scaled to the in-plane maximum detected intensity $I_{SmaxInplane}$, at the time of flight TOFsmax corresponding to the computed lateral position $LAP_{TOFSmax, \theta IPA}$. A qualitative indication of the out-of-plane distance may subsequently be indicated in reconstructed ultrasound image RUI. For example, an icon may be displayed that has a size that varies in accordance with:

$$Size = k_1 + k_2 \cdot \left(1 - \frac{I_{Smax}}{I_{SmaxInplane}}\right) \qquad\qquad \text{Equation 1}$$

and wherein $k_1$ and $k_2$ are constants and $k_1$ may include zero.

In another exemplary implementation, with reference to Fig. 3, the color of an icon may be configured to change based on the value of the maximum detected intensity $I_{Smax}$ in relation to $I_{SmaxInplane}$, at the time of flight TOFsmax.

An issue that has been discovered by the inventors of the present invention is however that the so-determined position may be susceptible to errors. The determined position may for example be influenced by the presence of high levels of noise or interference. The determined position may likewise be influenced when the maximum detected intensity $I_{Smax}$ or its signal to noise ratio or its signal to interference ratio is low. Another indicator of the possibility of an incorrectly determined position is a high rate of change of the determined position. In order to mitigate such errors, in the present invention position determination unit PDU suppresses the indication of the computed position $LAP_{TOFSmax, \theta IPA}$ if at least one of the following conditions are met:

a rate of movement of the computed position $LAP_{TOFSmax, \theta IPA}$ exceeds a first predetermined rate $R_{Max1}$;
an interference signal in the ultrasound signals exceeds a first predetermined value $Int_{Threshold1}$.

Thus, with reference to exemplary Fig. 3, if one of the aforementioned conditions are met, position determination unit PDU may cease to display circle $C_1$.

In summary, and with reference to Fig. 1, Fig. 2, Fig. 3 and Fig. 6, a system 10 for determining a position of an interventional device 11 respective an image plane 12 defined by an ultrasound imaging probe 13 of a beamforming ultrasound imaging system 14 in which the position of the interventional device 11 is determined based on ultrasound signals transmitted between the ultrasound imaging probe 13 and an ultrasound transducer 15 attached to the interventional device 11, includes:

image reconstruction unit IRU that provides reconstructed ultrasound image RUI corresponding to image plane 12 defined by ultrasound imaging probe 13; and
position determination unit PDU that:

computes a position $LAP_{TOFSmax, \theta IPA}$ of ultrasound transducer 15 respective image plane 12 based on a time of flight TOFsmax of a maximum detected intensity $I_{Smax}$ ultrasound signal transmitted between ultrasound imaging probe 13 and ultrasound transducer 15;
indicates the computed position $LAP_{TOFSmax, \theta IPA}$ in the reconstructed ultrasound image RUI; and
suppresses the indication of the computed position $LAP_{TOFSmax, \theta IPA}$ if at least one of the following conditions are met:

a rate of change of the computed position $LAP_{TOFSmax, \theta IPA}$ exceeds a first predetermined rate $R_{Max1}$;
an interference signal in the ultrasound signals exceeds a first predetermined value $Int_{Threshold1}$.

By so suppressing the indication of the computed position, it is prevented that a potentially inaccurate position is indicated.

The aforementioned conditions, i.e. the rate of change of the computed position, the interference signal in the ultrasound signals, the maximum detected intensity Ismax, the signal to noise ratio or the signal to interference ratio of the maximum detected intensity $I_{Smax}$ may be measured by hardware or a software-controlled processor or a combination of hardware and a software-controlled processor. The position determination unit that decides and implements the result of testing the condition is preferably implemented by a software-controlled processor.

In respect of determining the rate of change of the computed position $LAP_{TOFSmax, \theta IPA}$, position determination unit PDU which calculates position $LAP_{TOFSmax, \theta IPA}$ may determine, in a suitable coordinate space, the rate of change of the position. The rate of change may for instance include an angular rate of change of in-plane angle $\theta_{IPA}$ and/ or a rate of change of time of flight of the maximum detected ultrasound signal TOFsmax. Alternatively the polar coordinates of the computed position may be converted into Cartesian coordinate space in order to determine the rate of change. First predetermined rate $R_{Max1}$ may be set based on the likely rate that a user might be expected to move an interventional device within image plane 12. By way of an example, it may be considered unlikely that an operator would move interventional device 11 by e.g. 45 degrees at a time of flight corresponding to 10 centimeters within one image frame at say 100 frames per second. Such a threshold may be used to set first predetermined rate $R_{Max1}$. When position determination unit indicates a rate of change of position that exceeds this rate, the indication of the position, e.g. the provision of circle $C_1$ in reconstructed ultrasound image RUI in Fig. 3, may be inhibited.

In respect of the interference signal in the ultrasound signals, the measurement technique depends on whether ultrasound transducer 15 is a transmitter of a detector. When ultrasound transducer 15 is a detector an electrical circuit may for example be used to determine the root mean square, rms, value of the interference and/ or noise, in the electrical signals generated by the detector, or alternatively an analogue to digital converter may be used to sample these electrical signals and thus determine this value. Various hardware or software filters may be used to measure the noise and/ or interference within a predetermined bandwidth, and/ or to separate the noise and/ or interference from concurrent ultrasound signals. When ultrasound transducer 15 is a transmitter and it is desired to determine interference signals in the detected ultrasound signals, this may be carried out by analyzing the signals detected by ultrasound imaging probe 13. Processor-implemented position determination unit PDU may determine the noise and/ or interference from the digitized signals detected by ultrasound imaging probe 13 using equivalent software methods. Predetermined value $Int_{Threshold1}$ may be set e.g. based on typical measured or expected signal levels.

In some exemplary implementations position determination unit PDU may continue suppressing the indication of the computed position $LAP_{TOFSmax, \theta IPA}$ until at least one of the following corresponding conditions have been satisfied for a predetermined period:

a rate of change of the computed position $LAP_{TOFSmax, \theta IPA}$ is less than a second predetermined rate $R_{Max2}$;
an interference signal in the ultrasound signals is less than a second predetermined value $Int_{Threshold2}$.

By continuing to suppress the indication of the computed position until the corresponding condition is met for a predetermined period, the reliability of the system is further improved because it is ensured that the computed position is not indicated again until the position has stabilized over time.

The predetermined period may for instance be measured in seconds or fractions of a second, or in an integer number of frames or image lines. Synchronization to the frame rate by means of the latter has the advantage of less complex implementation.

Moreover, whilst the corresponding rates, values, thresholds and levels that continue suppression of the indication of the position may differ from those triggering the suppression, in some implementations the first predetermined rate $R_{Max1}$ is equal to the second predetermined rate $R_{Max2}$; and in some implementations the first predetermined value $Int_{Threshold1}$ is equal to the second predetermined value $Int_{Threshold2}$. The use of the same corresponding rates, values, thresholds and levels facilitates a less complex implementation. The use of a different threshold adds hysteresis into the decision making. In so doing more reliable system is provided.

With reference to Fig. 4, which illustrates a succession of periodic updates, RUT', RUI" to reconstructed ultrasound image RUI, in some exemplary implementations position determination unit PDU is configured to suppress the indication of the computed position $LAP_{TOFSmax, \theta IPA}$ based on an interference signal in the ultrasound signals exceeding a first predetermined value $Int_{Threshold1}$. The magnitude of the interference signal may be determined as described above. Moreover, in such implementations image reconstruction unit IRU is configured to periodically update the reconstructed ultrasound image RUI; the ultrasound signals corresponding to each reconstructed ultrasound image RUI being transmitted and detected by the ultrasound imaging probe 13 during a corresponding imaging frame period Tf. Position determination unit PDU is configured to determine the interference signal in the ultrasound signals between consecutive imaging frame periods.

Throughout Fig. 4B and 4C, updated reconstructed ultrasound images RUI' and RUI" indicate the progression of position-indicating circle $C_1$ horizontally and to the right of the figures. Fig. 4D indicates the corresponding imaging signals from beamforming ultrasound imaging probe 12, which are transmitted and detected within consecutive frames F, F' and F", each having a period Tf. Between consecutive frame periods there is a "quiet" period indicated by the vertical arrow in Fig. 4D during which negligible ultrasound signals are transmitted by the ultrasound imaging probe and no reflected ultrasound signals are expected to be detected. Consequently this quiet period represents a time when only the interference and/ or noise detected by the system can be reliably measured. The interference and/ or noise may exemplarily be measured between every frame, or between consecutive frames every N frames where N is an integer, or between consecutive frames after a random number of frames.

With reference to Fig. 5, which illustrates a reconstructed ultrasound image RUI that includes multiple image lines $L_{1..n}$, each line corresponding to a time of flight or depth dimension in the ultrasound image, in some exemplary implementations position determination unit PDU is configured to suppress the indication of the computed position $LAP_{TOFSmax, \theta IPA}$ based on an interference signal in the ultrasound signals exceeding a first predetermined value $Int_{Threshold1}$. In such implementations reconstructed ultrasound image RUI comprises a plurality of image lines $L_{1..n}$, each line corresponding to a depth dimension in the ultrasound image; and wherein the ultrasound signals corresponding to each line of the reconstructed ultrasound image RUI are transmitted and detected by the ultrasound imaging probe 13 during a corresponding image line period $T_1$. Position determination unit PDU is configured to determine the interference signal in the ultrasound signals between consecutive image line periods.

Ultrasound signals corresponding to image lines are typically transmitted in the consecutive manner illustrated in Fig. 5, and include, within image line period $T_1$, a transmit phase and a receive phase during which the ultrasound imaging probe transmits ultrasound signals and subsequently receives reflected ultrasound signals. Between the end of the receive phase of one imaging line and the transmit phase of the subsequent image line, there is a "quiet" period indicated by the vertical arrow in Fig. 5B and in which no reflections are expected. Consequently this quiet period represents a time when only the interference and/ or noise detected by the system can be reliably measured. The interference and/ or noise may exemplarily be measured between every line, or between consecutive lines after every M lines where M is an integer, or between consecutive lines after a random number of lines, or between the same two lines in consecutive or every N image frames.

With reference to Fig. 6, which illustrates a model MO describing an expected variation of in-plane maximum detected intensity, $I_{SmaxInplane}$ (dB) with time of flight, TOF, in some exemplary implementations, indicating the computed position $LAP_{TOFSmax, \theta IPA}$ in the reconstructed ultrasound image RUI may also include:

> computing an out-of-plane distance $D_{op}$ between the ultrasound transducer 15 and the image plane 12 by comparing the maximum detected intensity $I_{Smax}$ with a model MO describing an expected variation of in-plane maximum detected intensity $I_{SmaxInplane}$ with time of flight, at the time of flight $TOF_{Smax}$ of the ultrasound signal having the maximum detected intensity $I_{Smax}$; and
> indicating the out-of-plane distance $D_{op}$ in the reconstructed ultrasound image RUI.

As mentioned above an indication of the out-of-plane distance $D_{op}$ may be obtained through such a comparison. Comparing the maximum detected intensity $I_{Smax}$ with model MO may for instance involve determining a difference or ratio between detected intensity Ismax and the in-plane maximum detected intensity, $I_{SmaxInplane}$. The maximum detected intensity $I_{Smax}$ at the computed lateral position $LAP_{TOFSmax,\,\theta IPA}$ of the ultrasound transducer may thus be scaled to the in-plane maximum detected intensity $I_{SmaxInplane}$. By using model MO, a qualitative indication of out of plane distance $D_{op}$ may be obtained with low computational effort. Moreover, the problem of computing a potentially inaccurate position may be particularly acute at large out-of-plane distances where detected ultrasound signals from the ultrasound imaging probe are low. Thus suppressing the indicated position under the aforementioned conditions, such as the illustrated maximum detected intensity $I_{SmaxThreshold1}$ which is here exemplarily expressed as a proportion of the in-plane maximum intensity value, may be particularly beneficial in implementations in which the interventional device is routinely disposed in an out-of-plane position.

With reference to Fig. 7, which illustrates a succession of reconstructed ultrasound images RUI in which an out-of-plane distance $D_{op}$ is indicated by means of a first icon $C_{op}$ at the computed lateral position $LAP_{TOFSmax,\,\theta IPA}$ in these exemplary implementations, indicating out-of-plane distance $D_{op}$ may comprise providing a first icon $C_{op}$ at the computed lateral position $LAP_{TOFSmax,\,\theta IPA}$, the first icon $C_{op}$ being indicative of a circular zone with a radius corresponding to the out-of-plane distance $D_{op}$. In Fig. 7 the lateral position of interventional device 11 in reconstructed ultrasound images RUI, RUI' and RUI" remains constant respective image plane 12 and its out-of-plane distance $D_{op}$ is reduced in positions LAP' and LAP" in Fig. 7B and Fig. 7C. Consequently the corresponding radius of circles $C_{op}$' and $C_{op}$" is reduced. Optionally, the radius of first icon $C_{op}$ may be determined by scaling the maximum detected intensity Ismax to the expected in-plane maximum detected intensity $I_{SmaxInplane}$, at the time of flight $TOF_{Smax}$ of the ultrasound signal having the maximum detected intensity Ismax.

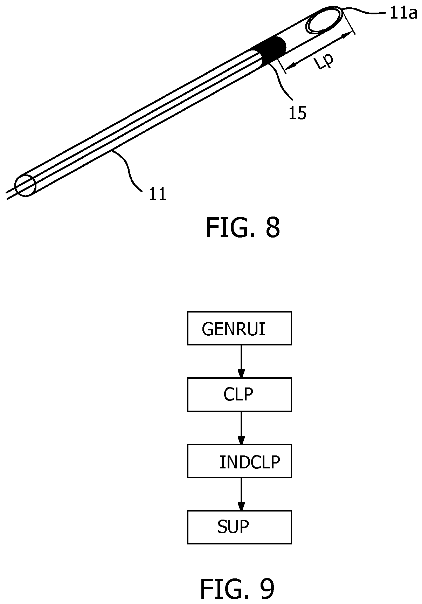

Fig. 8 illustrates an interventional device 11 that is suitable for use with system 10. Ultrasound transducer 15 may be attached at a predetermined distance $L_p$ from a feature, e.g. distal end 11a of interventional device 11. Ultrasound transducer 15 may be attached to interventional device 11 by various means including using an adhesive. Electrical conductors that carry electrical signals from ultrasound transducer 11 to position determination unit PDU are also shown, although as mentioned above it is contemplated to alternatively use a wireless link to communicate the transducer signals with position determination unit PDU.

Ultrasound transducer 15 described above with particular reference to Fig. 1, Fig. 2 and Fig. 8 may be provided by a variety of piezoelectric materials. Both hard and soft piezoelectric materials are suitable. Micromachined Electromechanical Structures, i.e. MEMS devices such as Capacitive Micromachined Ultrasound Transducers, i.e. CMUT, devices are also suitable. When the ultrasound transducer is a detector, preferably it is formed from Polyvinylidene fluoride, otherwise known as PVDF whose mechanical properties and manufacturing processes lend themselves to attachment to curved surfaces such as medical needles. Alternative materials include a PVDF co-polymer such as polyvinylidene fluoride trifluoroethylene, a PVDF ter-polymer such as P(VDF-TrFE-CTFE). Preferably the ultrasound transducer is wrapped around an axis of the interventional device in order to provide sensing around 360 degrees of rotation about the axis although this need not always be the case.

Fig. 9 illustrates various method steps of a method that may be used with system 10. With reference to Fig. 9 a method of determining a position of interventional device 11 respective image plane 12 defined by ultrasound imaging probe 13 of beamforming ultrasound imaging system 14 in which the position of interventional device 11 is determined based on ultrasound signals transmitted between ultrasound imaging probe 13 and ultrasound transducer 15 attached to interventional device 11; includes the steps of:

> generating GENRUI a reconstructed ultrasound image RUI corresponding to an image plane 12 defined by the ultrasound imaging probe 13;
> computing CLP a lateral position $LAP_{TOFSmax,\,\theta IPA}$ of the ultrasound transducer 15 respective the image plane 12 based on a time of flight TOFsmax of a maximum detected intensity $I_{Smax}$ ultrasound signal transmitted between the ultrasound imaging probe 13 and the ultrasound transducer 15;
> indicating INDCLP the computed position $LAP_{TOFSmax,\,\theta IPA}$ in the reconstructed ultrasound image RUI; and
> suppressing SUP the indication of the computed position $LAP_{TOFSmax,\,\theta IPA}$ if at least one of the following conditions are met:

>> a rate of change of the computed position $LAP_{TOFSmax,\,\theta IPA}$ exceeds a first predetermined rate $R_{Max1}$;
>> an interference signal in the ultrasound signals exceeds a first predetermined value $Int_{Threshold1}$.

The method may optionally include the step of:
computing an out-of-plane distance $D_{op}$ between the ultrasound transducer 15 and the image plane 12 by comparing the maximum detected intensity $I_{Smax}$ with a model MO describing an expected variation of in-plane maximum detected intensity $I_{SmaxInplane}$ with time of flight, at the time of flight $TOF_{Smax}$ of the ultrasound signal having the maximum detected intensity $I_{Smax}$.

The step of indicating INDCLP the computed position $LAP_{TOFSmax, \theta IPA}$ in the reconstructed ultrasound image RUI may optionally include:

indicating the out-of-plane distance $D_{op}$ in the reconstructed ultrasound image RUI.

It is to be noted that other implementations of the method may additionally incorporate one or more aspects described with respect to an implementation of the system.

The method steps illustrated in Fig. 9, optionally including other method steps described herein, may be stored on a computer program product as instructions that are executable by a processor. The computer program product may be provided by dedicated hardware, or hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

In this respect a computer program product is also provided for use with system 10. The computer program product includes instructions which when executed on a processor of system 10 for determining a position of an interventional device 11 respective an image plane 12 defined by an ultrasound imaging probe 13 of a beamforming ultrasound imaging system 14 in which the position of the interventional device 11 is determined based on ultrasound signals transmitted between the ultrasound imaging probe 13 and an ultrasound transducer 15 attached to the interventional device 11; cause the processor to carry out the aforementioned method steps.

In summary a system has been described for determining a position of an interventional device respective an image plane defined by an ultrasound imaging probe of a beamforming ultrasound imaging system in which the position of the interventional device is determined based on ultrasound signals transmitted between the ultrasound imaging probe and an ultrasound transducer attached to the interventional device. The system includes an image reconstruction unit and a position determination unit. The image reconstruction unit provides a reconstructed ultrasound image corresponding to an image plane defined by the ultrasound imaging probe. The position determination unit computes a position of the ultrasound transducer respective the image plane based on a time of flight of a maximum detected intensity ultrasound signal transmitted between the ultrasound imaging probe and the ultrasound transducer. The position determination unit also indicates the computed position in the reconstructed ultrasound image. Moreover, the position determination unit suppresses the indication of the computed position if at least one of the following conditions are met:

a rate of change of the computed position exceeds a first predetermined rate;
an interference signal in the ultrasound signals exceeds a first predetermined value.

Whilst the invention has been illustrated and described in detail in the drawings and foregoing description in relation to a medical needle, such illustrations and descriptions are to be considered illustrative or exemplary and not restrictive. Any reference signs in the claims should not be construed as limiting the scope of the invention. Moreover it is to be understood that the various examples, implementations and embodiments illustrated herein may be combined in order to provide various systems and methods for determining a position of an interventional device respective an image plane of a beamforming ultrasound imaging system.

**Claims**

1. System (10) for determining a position of an interventional device (11) respective an image plane (12) defined by an ultrasound imaging probe (13) of a beamforming ultrasound imaging system (14) in which the position of the interventional device (11) is determined based on ultrasound signals transmitted between the ultrasound imaging probe (13) and an ultrasound transducer (15) attached to the interventional device (11), the system (10) comprising:

an image reconstruction unit (IRU) configured to provide a reconstructed ultrasound image (RUI) corresponding to an image plane (12) defined by the ultrasound imaging probe (13);
a position determination unit (PDU) configured to:

compute a position ($LAP_{TOFSmax, \theta IPA}$) of the ultrasound transducer (15) respective the image plane (12) based on a time of flight ($TOFsmax$) of a maximum detected intensity ($I_{Smax}$) ultrasound signal transmitted between the ultrasound imaging probe (13) and the ultrasound transducer (15); and to
indicate the computed position ($LAP_{TOFSmax, \theta IPA}$) in the reconstructed ultrasound image (RUI); and **characterised in that** the position determination unit (PDU) is further configured to suppress the indication of the computed position ($LAP_{TOFSmax, \theta IPA}$) if at least one of the following conditions are met:

a rate of change of the computed position ($LA\text{-}P_{TOFSmax, \theta IPA}$) exceeds a first predetermined rate ($R_{Max1}$);
an interference signal in the ultrasound signals exceeds a first predetermined value ($Int_{Threshold1}$).

2. The system (10) according to claim 1 wherein the position determination unit (PDU) is further configured to continue suppressing the indication of the computed position ($LAP_{TOFSmax, \theta IPA}$) until at least one of the following corresponding conditions have been satisfied for a predetermined period:

a rate of change of the computed position ($LAP_{TOFSmax, \theta IPA}$) is less than a second predetermined rate ($R_{Max2}$);
an interference signal in the ultrasound signals is less than a second predetermined value ($Int_{Threshold2}$).

3. The system (10) according to claim 2 wherein the first predetermined rate ($R_{Max1}$) is equal to the second predetermined rate ($R_{Max2}$); or wherein the first predetermined value ($Int_{Threshold1}$) is equal to the second predetermined value ($Int_{Threshold2}$).

4. The system (10) according to any one of claims 1 - 3 wherein the position determination unit (PDU) is configured to suppress the indication of the computed position ($LAP_{TOFSmax, \theta IPA}$) based on an interference signal in the ultrasound signals exceeding a first predetermined value ($Int_{Threshold1}$); and wherein the image reconstruction unit (IRU) is configured to periodically update the reconstructed ultrasound image (RUI); the ultrasound signals corresponding to each reconstructed ultrasound image (RUI) being transmitted and detected by the ultrasound imaging probe (13) during a corresponding imaging frame period ($Tf$); and wherein the position determination unit (PDU) is configured to determine the interference signal in the ultrasound signals between consecutive imaging frame periods.

5. The system (10) according to any one of claims 1 - 3 wherein the position determination unit (PDU) is configured to suppress the indication of the computed position ($LAP_{TOFSmax, \theta IPA}$) based on an interference signal in the ultrasound signals exceeding a first predetermined value ($Int_{Threshold1}$); and wherein the reconstructed ultrasound image (RUI) comprises a plurality of image lines ($L_{1..n}$), each line corresponding to a depth dimension in the ultrasound image; and wherein the ultrasound signals corresponding to each line of the reconstructed ultrasound image (RUI) are transmitted and detected by the ultrasound imaging probe (13) during a corresponding image line period ($T_1$); and wherein the position determination unit (PDU) is configured to determine the interference signal in the ultrasound signals between consecutive image line periods.

6. The system (10) according to any previous claim wherein indicating the computed position ($LA\text{-}P_{TOFSmax, \theta IPA}$) in the reconstructed ultrasound image (RUI) includes:

computing an out-of-plane distance ($D_{op}$) between the ultrasound transducer (15) and the image plane (12) by comparing the maximum detected intensity ($Ismax$) with a model (MO) describing an expected variation of in-plane maximum detected intensity ($I_{SmaxInplane}$) with time of flight, at the time of flight ($TOF_{Smax}$) of the ultrasound signal having the maximum detected intensity ($Ismax$); and
indicating the out-of-plane distance ($D_{op}$) in the reconstructed ultrasound image (RUI).

7. The system (10) according to claim 6 wherein indicating the out-of-plane distance ($D_{op}$) comprises providing a first icon ($C_{op}$) at the computed lateral position ($LA\text{-}P_{TOFSmax, \theta IPA}$), the first icon ($C_{op}$) being indicative of a circular zone with a radius corresponding to the out-of-plane distance ($D_{op}$).

8. The system (10) according to claim 7 wherein the radius is determined based on scaling the maximum detected intensity ($Ismax$) to the expected in-plane maximum detected intensity ($I_{SmaxInplane}$), at the time of flight ($TOF_{Smax}$)

of the ultrasound signal having the maximum detected intensity (Ismax).

9. The system (10) according to any previous claim further comprising an interventional device (11) having an ultrasound transducer (15) attached thereto.

10. Method of determining a position of an interventional device (11) respective an image plane (12) defined by an ultrasound imaging probe (13) of a beamforming ultrasound imaging system (14) in which the position of the interventional device (11) is determined based on ultrasound signals transmitted between the ultrasound imaging probe (13) and an ultrasound transducer (15) attached to the interventional device (11); the method comprising the steps of:

generating (GENRUI) a reconstructed ultrasound image (RUI) corresponding to an image plane (12) defined by the ultrasound imaging probe (13);
computing (CLP) a lateral position ($LAP_{TOFSmax, \theta IPA}$) of the ultrasound transducer (15) respective the image plane (12) based on a time of flight (TOFsmax) of a maximum detected intensity ($I_{Smax}$) ultrasound signal transmitted between the ultrasound imaging probe (13) and the ultrasound transducer (15);
indicating (INDCLP) the computed position ($LAP_{TOFSmax, \theta IPA}$) in the reconstructed ultrasound image (RUI);
**characterised in that** the method further comprises the steps of:
suppressing (SUP) the indication of the computed position ($LAP_{TOFSmax, \theta IPA}$) if at least one of the following conditions are met:

a rate of change of the computed position ($LAP_{TOFSmax, \theta IPA}$) exceeds a first predetermined rate ($R_{Max1}$);
an interference signal in the ultrasound signals exceeds a first predetermined value ($Int_{Threshold1}$).

11. The method according to claim 10 further comprising:

computing an out-of-plane distance ($D_{op}$) between the ultrasound transducer (15) and the image plane (12) by comparing the maximum detected intensity (Ismax) with a model (MO) describing an expected variation of in-plane maximum detected intensity ($I_{SmaxInplane}$) with time of flight, at the time of flight ($TOF_{Smax}$) of the ultrasound signal having the maximum detected intensity (Ismax); and
wherein the step of indicating (INDCLP) the computed position ($LAP_{TOFSmax, \theta IPA}$) in the reconstructed ultrasound image (RUI) further comprises:
indicating the out-of-plane distance ($D_{op}$) in the reconstructed ultrasound image (RUI).

12. Computer program product comprising instructions which when executed on a processor of a system (10) for determining a position of an interventional device (11) respective an image plane (12) defined by an ultrasound imaging probe (13) of a beamforming ultrasound imaging system (14) in which the position of the interventional device (11) is determined based on ultrasound signals transmitted between the ultrasound imaging probe (13) and an ultrasound transducer (15) attached to the interventional device (11) cause the processor to carry out the method steps of claim 10 or 11.

**Patentansprüche**

1. System (10) zur Bestimmung der Position einer Eingriffsvorrichtung (11) bzw. einer Bildebene (12), die durch eine Ultraschallabbildungssonde (13) eines Strahlformungs-Ultraschallabbildungssystems (14) definiert ist, in dem die Position der Eingriffsvorrichtung (11) auf der Grundlage von Ultraschallsignalen bestimmt wird, die zwischen der Ultraschallabbildungssonde (13) und einem an der Eingriffsvorrichtung (11) angebrachten Ultraschallwandler (15) übertragen werden, wobei das System (10) folgendes umfasst:

eine Bildrekonstruktionseinheit (IRU), die so konfiguriert ist, dass sie ein rekonstruiertes Ultraschallbild (RUI) liefert, das einer Bildebene (12) entspricht, die durch die Ultraschallabbildungssonde (13) definiert ist;
eine Positionsbestimmungseinheit (PDU), die dafür konfiguriert ist:

eine Position ($LAP_{TOFSmax, \Theta IPA}$) des Ultraschallwandlers (15) zu berechnen bzw. der Bildebene (12) auf der Grundlage einer Flugzeit ($TOF_{smax}$) eines Ultraschallsignals mit maximaler detektierter Intensität ($I_{Smax}$), das zwischen der bildgebenden Ultraschallsonde (13) und dem Ultraschallwandler (15) übertragen wird; und um die berechnete Position ($LAP_{TOFSmax, \theta iPA}$) in dem rekonstruierten Ultraschallbild (RUI) anzugeben; und

**dadurch gekennzeichnet, dass** die Positionsbestimmungseinheit (PDU) außerdem so konfiguriert ist, dass sie die Angabe der berechneten Position ($LAP_{TOFSmax, \Theta IPA}$) unterdrückt wird, wenn mindestens eine der folgenden Bedingungen erfüllt ist:

eine Änderungsrate der berechneten Position ($LAP_{TOFSmax, \Theta IPA}$) übersteigt eine erste vorbestimmte Rate ($R_{Max1}$) ;
ein Störsignal in den Ultraschallsignalen einen ersten vorgegebenen Wert ($Int_{Schwellenwert1}$) überschreitet.

2. System (10) nach Anspruch 1, wobei die Positionsbestimmungseinheit (PDU) ferner so konfiguriert ist, dass die Anzeige der berechneten Position ($LAP_{TOFSmax, \Theta IPA}$) so lange unterdrückt wird, bis mindestens eine der folgenden entsprechenden Bedingungen für einen vorgegebenen Zeitraum erfüllt ist:

eine Änderungsrate der berechneten Position ($LAP_{TOFSmax, \Theta IPA}$) ist kleiner ist als eine zweite vorbestimmte Rate ($R_{MAX2}$);
ein Störsignal in den Ultraschallsignalen ist kleiner als ein zweiter vorbestimmter Wert ($Int_{Schwellenwert2}$).

3. System (10) nach Anspruch 2, wobei die erste vorbestimmte Rate ($R_{Max1}$) gleich der zweiten vorgegebenen Rate ($R_{max2}$) ist; oder wobei der erste vorgegebene Wert ($Int_{Schwellenwert1}$) gleich dem zweiten vorgegebenen Wert ($Int_{Schwellenwert2}$) ist.

4. Das System (10) nach einem der Ansprüche 1 bis 3, wobei die Position Bestimmungseinheit (PDU) so konfiguriert ist, dass sie die Anzeige der berechneten Position ($LAP_{TOFSmax, \Theta IPA}$) auf der Grundlage eines Interferenzsignals in den Ultraschallsignalen unterdrückt, das einen ersten vorbestimmten Wert ($Int_{Schwellenwert1}$) überschreitet; und wobei die Bildrekonstruktionseinheit (IRU) so konfiguriert ist, dass sie das rekonstruierte Ultraschallbild (RUI) periodisch aktualisiert; wobei die Ultraschallsignale, die jedem rekonstruierten Ultraschallbild (RUI) entsprechen, von der Ultraschallabbildungssonde (13) während einer entsprechenden Abbildungsrahmenperiode ($T_f$) übertragen und erfasst werden; und wobei die Positionsbestimmungseinheit (PDU) so konfiguriert ist, dass sie das Interferenzsignal in den Ultraschallsignalen zwischen aufeinanderfolgenden Abbildungsrahmenperioden bestimmt.

5. Das System (10) nach einem der Ansprüche 1 bis 3, wobei die Position Bestimmungseinheit (PDU) so konfiguriert ist, dass sie die Anzeige der berechneten Position ($LAP_{TOFSmax, \Theta iPA}$) auf der Grundlage eines Interferenzsignals in den Ultraschallsignalen, das einen ersten vorbestimmten Wert ($Int_{Schwellenwert1}$) überschreitet, unterdrückt; und wobei das rekonstruierte Ultraschallbild (RUI) eine Vielzahl von Bildzeilen ($L_{1..n}$) umfasst, wobei jede Zeile einer Tiefendimension in dem Ultraschallbild entspricht; und wobei die Ultraschallsignale, die jeder Zeile des rekonstruierten Ultraschallbildes (RUI) entsprechen, von der Ultraschallabbildungssonde (13) während einer entsprechenden Bildzeilenperiode ($T_1$) übertragen und erfasst werden; und wobei die Positionsbestimmungseinheit (PDU) so konfiguriert ist, dass sie das Interferenzsignal in den Ultraschallsignalen zwischen aufeinanderfolgenden Bildzeilenperioden bestimmt.

6. System (10) nach einem der vorhergehenden Ansprüche, wobei die Angabe der berechneten Position ($LAP_{TOFSmax, \Theta IPA}$) in dem rekonstruierten Ultraschallbild (RUI) Folgendes umfasst: Berechnen eines Abstandes ($D_{op}$) außerhalb der Ebene zwischen dem Ultraschallwandler (15) und der Bildebene (12) durch Vergleichen der maximalen erfassten Intensität ($I_{Smax}$) mit einem Modell (MO), das eine erwartete Variation der maximalen erfassten Intensität ($I_{SmaxInebene}$) in der Ebene mit der Flugzeit (TOFsmax) des Ultraschallsignals mit der maximalen erfassten Intensität (Ismax) beschreibt; und
die den Abstand außerhalb der Ebene ($D_{op}$) im rekonstruierten Ultraschallbild (RUI) angibt.

7. System (10) nach Anspruch 6, bei dem die Anzeige des Abstand außerhalb der Ebene ($D_{op}$) das Bereitstellen eines ersten Symbols ($C_{op}$) an der berechneten seitlichen Position ($LAP_{TOFSmax, \Theta IPA}$) umfasst, wobei das erste Symbol ($C_{op}$) eine kreisförmige Zone mit einem Radius anzeigt, der dem Abstand außerhalb der Ebene ($D_{op}$) entspricht.

8. System (10) nach Anspruch 7, wobei der Radius bestimmt wird auf der Grundlage bei der Skalierung der maximalen detektierten Intensität ($I_{Smax}$) auf die erwartete maximale detektierte Intensität ($I_{SmaxInebene}$) in der Ebene zum Flugzeitpunkt (TOFsmax) des Ultraschallsignals mit der maximalen detektierten Intensität ($I_{Smax}$).

9. Das System (10) nach einem der vorhergehenden Ansprüche umfasst ferner ein Interventionsgerät (11) mit einem daran befestigten Ultraschallwandler (15).

**10.** Verfahren zur Bestimmung der Position einer Eingriffsvorrichtung (11) bzw. eine Bildebene (12), die durch eine Ultraschallabbildungssonde (13) eines Strahlformungs-Ultraschallabbildungssystems (14) definiert ist, in dem die Position der Eingriffsvorrichtung (11) auf der Grundlage von Ultraschallsignalen bestimmt wird, die zwischen der Ultraschallabbildungssonde (13) und einem an der Eingriffsvorrichtung (11) angebrachten Ultraschallwandler (15) übertragen werden; wobei das Verfahren die folgenden Schritte umfasst:

Erzeugen (GENRUI) eines rekonstruierten Ultraschallbildes (RUI), das einer durch den Ultraschall-Bildgeber (13) definierten Bildebene (12) entspricht;
Berechnung (CLP) einer seitlichen Position ($LAP_{TOFSmax, \Theta IPA}$) des Ultraschallwandlers (15) bzw. der Bildebene (12) auf der Grundlage einer Flugzeit (TOFsmax) eines Ultraschallsignals mit maximaler erfasster Intensität (Ismax), das zwischen der bildgebenden Ultraschallsonde (13) und dem Ultraschallwandler (15) übertragen wird;
Angabe (INDCLP) der berechneten Position ($LAP_{TOFSmax, \Theta IPA}$) in dem rekonstruierten Ultraschallbild (RUI);
**dadurch gekennzeichnet, dass** das Verfahren ferner die folgenden Schritte umfasst:
Unterdrückung (SUP) der Angabe der berechneten Position ($LAP_{TOFSmax, \Theta IPA}$), wenn mindestens eine der folgenden Bedingungen erfüllt ist:

eine Änderungsrate der berechneten Position ($LAP_{TOFSmax, \Theta IPA}$) eine erste vorbestimmte Rate ($R_{Max1}$) überschreitet;
ein Störsignal in den Ultraschallsignalen einen ersten vorgegebenen Wert ($Int_{Schwellenwert1}$) überschreitet.

**11.** Das Verfahren nach Anspruch 10 umfasst ferner:

Berechnen eines Abstandes ($D_{op}$) außerhalb der Ebene zwischen dem Ultraschallwandler (15) und der Bildebene (12) durch Vergleichen der maximalen erfassten Intensität ($I_{Smax}$) mit einem Modell (MO), das eine erwartete Variation der maximalen erfassten Intensität ($I_{SmaxInebene}$) in der Ebene mit der Flugzeit (TOFsmax) des Ultraschallsignals mit der maximalen erfassten Intensität (Ismax) beschreibt; und
wobei der Schritt des Anzeigens (INDCLP) der berechneten Position ($LAP_{TOFSmax, \Theta IPA}$) in dem rekonstruierten Ultraschallbild (RUI) ferner weiteres umfasst;
Angabe des Abstands außerhalb der Ebene ($D_{op}$) im rekonstruierten Ultraschallbild (RUI).

**12.** Computerprogrammprodukt das Anweisungen beinhaltet, die bei Ausführung auf einem Prozessor eines Systems (10) zum Bestimmen einer Position einer Eingriffsvorrichtung (11) bzw. einer Bildebene (12), die durch eine Ultraschallabbildungssonde (13) eines Strahlformungs-Ultraschallabbildungssystems (14) definiert ist, in dem die Position der Eingriffsvorrichtung (11) auf der Grundlage von Ultraschallsignalen bestimmt wird, die zwischen der Ultraschallabbildungssonde (13) und einem an der Eingriffsvorrichtung (11) angebrachten Ultraschallwandler (15) übertragen werden, den Prozessor veranlassen, die Verfahrensschritte nach Anspruch 10 oder 11 auszuführen

## Revendications

**1.** Système (10) pour déterminer la position d'un dispositif d'intervention (11) par rapport à un plan d'image (12) défini par une sonde d'imagerie à ultrasons (13) d'un système d'imagerie à ultrasons à formation de faisceau (14) dans lequel la position du dispositif d'intervention (11) est déterminée sur la base de signaux ultrasonores transmis entre la sonde d'imagerie à ultrasons (13) et un transducteur à ultrasons (15) fixé au dispositif d'intervention (11), le système (10) comprenant:

une unité de reconstruction d'image (IRU) configurée pour fournir une image ultrasonore reconstruite (RUI) correspondant à un plan image (12) défini par la sonde d'imagerie ultrasonore (13);
une unité de détermination de position (PDU) configurée pour:

calculer une position ($LAP_{TOFSmax, \Theta IPA}$) du transducteur à ultrasons (15) par rapport au plan image (12) sur la base d'un temps de vol ($TOF_{Smax}$) d'un signal ultrasonore d'intensité maximale détectée (Ismax) transmis entre la sonde d'imagerie ultrasonore (13) et le transducteur ultrasonore (15); et pour
indiquer la position calculée ($LA\text{-}P_{TOFSmax, \Theta IPA}$) dans l'image ultrasonore reconstruite (RUI); et
**caractérisé par le fait que** l'unité de détermination de position (PDU) est en outre configurée pour supprimer l'indication de la position calculée ($LA\text{-}P_{TOFSmax, \Theta IPA}$) si au moins une des conditions suivantes est remplie:

un taux de changement de la position calculée ($LA\text{-}P_{TOFSmax, \Theta IPA}$) dépasse un premier taux prédéterminé ($R_{Max1}$);
un signal d'interférence dans les signaux ultrasonores dépasse une première valeur prédéterminée ($Int_{Seuil1}$).

2. Système (10) selon la revendication 1, dans lequel l'unité de détermination de position (PDU) est en outre configurée pour continuer à supprimer l'indication de la position calculée (LA-P$_{TOFSmax, \Theta IPA}$) jusqu'à ce qu'au moins une des conditions correspondantes suivantes ait été satisfaite pendant une période prédéterminée:

un taux de changement de la position calculée (LA-P$_{TOFSmax, \Theta IPA}$) est inférieur à un second taux prédéterminé ($R_{MAx2}$);
un signal d'interférence dans les signaux ultrasonores est inférieur à une deuxième valeur prédéterminée ($Int_{Seuil2}$).

3. Système (10) selon la revendication 2, dans lequel le premier taux prédéterminé ($R_{MAX1}$) est égal au deuxième taux prédéterminé ($R_{MAX2}$) ; ou dans lequel la première valeur prédéterminée ($Int_{Seuil1}$) est égale à la deuxième valeur prédéterminée ($Int_{Seuil2}$).

4. Système (10) selon l'une quelconque des revendications 1 à 3, dans lequel l'unité de détermination de position (PDU) est configurée pour supprimer l'indication de la position calculée (LA-P$_{TOFSmax, \Theta IPA}$) sur la base d'un signal d'interférence dans les signaux ultrasonores dépassant une première valeur prédéterminée ($Int_{Seuil1}$); et dans lequel l'unité de reconstruction d'image (IRU) est configurée pour mettre à jour périodiquement l'image ultrasonore reconstruite (RUI); les signaux ultrasonores correspondant à chaque image ultrasonore reconstruite (RUI) étant transmis et détectés par la sonde d'imagerie ultrasonore (13) pendant une période de trame d'imagerie correspondante ($T_f$); et dans lequel l'unité de détermination de position (PDU) est configurée pour déterminer le signal d'interférence dans les signaux ultrasonores entre des périodes de trame d'imagerie consécutives.

5. Système (10) selon l'une quelconque des revendications 1 à 3, dans lequel l'unité de détermination de position (PDU) est configurée pour supprimer l'indication de la position calculée (LA-P$_{TOFSmax, \Theta IPA}$) sur la base d'un signal d'interférence dans les signaux ultrasonores dépassant une première valeur prédéterminée ($Int_{Seuil1}$); et dans lequel l'image ultrasonore reconstruite (RUI) comprend une pluralité de lignes d'image ($L_{1..n}$) chaque ligne correspondant à une dimension de profondeur dans l'image ultrasonore ; et dans lequel les signaux ultrasonores correspondant à chaque ligne de l'image ultrasonore reconstruite (RUI) sont transmis et détectés par la sonde d'imagerie ultrasonore (13) pendant une période de ligne d'image correspondante ($T_1$); et dans lequel l'unité de détermination de position (PDU) est configurée pour déterminer le signal d'interférence dans les signaux ultrasonores entre des périodes de ligne d'image consécutives.

6. Système (10) selon l'une quelconque des revendications précédentes, dans lequel l'indication de la position calculée (LAP$_{TOFSmax, \Theta IPA}$) dans l'image ultrasonore reconstruite (RUI) comprend: le calcul d'une distance hors plan ($D_{op}$) entre le transducteur à ultrasons (15) et le plan image (12) en comparant l'intensité maximale détectée ($I_{Smax}$) avec un modèle (MO) décrivant une variation attendue de l'intensité maximale détectée dans le plan ($I_{SmaxInplan}$) avec le temps de vol, au temps de vol ($TOF_{smax}$) du signal ultrasonore ayant l'intensité maximale détectée ($I_{Smax}$); et indiquant la distance hors du plan ($D_{op}$) dans l'image ultrasonore reconstruite (RUI).

7. Système (10) selon la revendication 6, dans lequel l'indication de la distance hors plan ($D_{op}$) comprend la fourniture d'une première icône ($C_{op}$) à la position latérale calculée (LA-P$_{TOFSmax, \Theta IPA}$) , la première icône ($C_{op}$) étant indicative d'une zone circulaire avec un rayon correspondant à la distance hors plan ($D_{op}$).

8. Système (10) selon la revendication 7, dans lequel le rayon est déterminé sur la base de la mise à l'échelle de l'intensité maximale détectée ($I_{Smax}$) à l'intensité maximale détectée dans le plan prévue ($I_{smaxInplan}$), au moment du vol ($TOF_{Smax}$) du signal ultrasonore ayant l'intensité maximale détectée ($I_{Smax}$).

9. Le système (10) selon l'une quelconque des revendications précédentes comprenant en outre un dispositif d'intervention (11) auquel est fixé un transducteur à ultrasons (15).

10. Procédé de détermination d'une position d'un dispositif d'intervention (11) par rapport à un plan d'image (12) défini par une sonde d'imagerie à ultrasons (13) d'un système d'imagerie à ultrasons à formation de faisceau (14) dans lequel la position du dispositif d'intervention (11) est déterminée sur la base de signaux ultrasonores transmis entre la sonde d'imagerie à ultrasons (13) et un transducteur à ultrasons (15) fixé au dispositif d'intervention (11); le procédé comprenant les étapes de:

génération (GENRUI) d'une image ultrasonore reconstruite (RUI) correspondant à un plan image (12) défini par la sonde d'imagerie ultrasonore (13);

calcul (CLP) d'une position latérale (LAP$_{TOFSmax, \Theta IPA}$) du transducteur à ultrasons (15) par rapport au plan image (12) sur la base d'un temps de vol (TOF$_{Smax}$) d'un signal ultrasonore d'intensité maximale détectée (I$_{Smax}$) transmis entre la sonde d'imagerie à ultrasons (13) et le transducteur à ultrasons (15);

indication (INDCLP) de la position calculée (LAP$_{TOFSmax, \Theta IPA}$) dans l'image ultrasonore reconstruite (RUI); **caractérisé en ce que** le procédé comprend en outre les étapes consistant à:

suppression (SUP) de l'indication de la position calculée (LA-P$_{TOFSmax, \Theta IPA}$) si au moins une des conditions suivantes est remplie:

un taux de changement de la position calculée (LA-P$_{TOFSmax, \Theta IPA}$) dépasse un premier taux prédéterminé (R$_{MAX1}$);

un signal d'interférence dans les signaux ultrasonores dépasse une première valeur prédéterminée (Int$_{TSeuil1}$).

**11.** Procédé selon la revendication 10 comprenant en outre:

le calcul d'une distance hors plan (D$_{op}$) entre le transducteur à ultrasons (15) et le plan image (12) en comparant l'intensité maximale détectée (I$_{Smax}$) avec un modèle (MO) décrivant une variation attendue de l'intensité maximale détectée dans le plan (I$_{SmaxInplan}$) avec le temps de vol, au temps de vol (TOF$_{Smax}$) du signal ultrasonore ayant l'intensité maximale détectée (I$_{Smax}$); et

dans lequel l'étape d'indication (INDCLP) de la position calculée (LAP$_{TOFSmax, \Theta IPA}$) dans l'image ultrasonore reconstruite (RUI) comprend en outre:
indiquant la distance hors du plan (D$_{op}$) dans l'image ultrasonore reconstruite (RUI).

**12.** Produit de programme informatique comprenant des instructions qui, lorsqu'elles sont exécutées sur un processeur d'un système (10) pour déterminer une position d'un dispositif d'intervention (11) respectif d'un plan d'image (12) défini par une sonde d'imagerie ultrasonore (13) d'un système d'imagerie ultrasonore à formation de faisceau (14) dans lequel la position du dispositif d'intervention (11) est déterminée sur la base de signaux ultrasonores transmis entre la sonde d'imagerie par ultrasons (13) et un transducteur à ultrasons (15) fixé au dispositif d'intervention (11) amène le processeur à effectuer les étapes de procédé de la revendication 10 ou 11.

FIG. 1

EP 3 833 265 B1

FIG. 2

FIG. 3

FIG. 4A          FIG. 4B          FIG. 4C

FIG. 4D

FIG. 5A

FIG. 5B

FIG. 6

FIG. 7A

FIG. 7B

FIG. 7C

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018060499 A1
- US 20160324501 A1
- WO 2011138698 A1
- WO 2015101949 A1
- WO 2016009350 A1